(19) Europäisches Patentamt
European Patent Office
Office européen des brevets

(11) Veröffentlichungsnummer : **0 095 611**
**B1**

(12) # EUROPÄISCHE PATENTSCHRIFT

(45) Veröffentlichungstag der Patentschrift : **28.11.84**

(51) Int. Cl.³ : **C 07 C 43/188**, C 07 C 43/184, C 07 C 41/01, C 07 C 41/20

(21) Anmeldenummer : **83104575.2**

(22) Anmeldetag : **10.05.83**

(54) **2-Methoxyethyl-cyclododecenylether, Verfahren zu seiner Herstellung sowie seine Verwendung zur Herstellung von 2-Methoxyethyl-cyclododecylether.**

(30) Priorität : **27.05.82 DE 3219915**

(43) Veröffentlichungstag der Anmeldung : **07.12.83 Patentblatt 83/49**

(45) Bekanntmachung des Hinweises auf die Patenterteilung : **28.11.84 Patentblatt 84/48**

(84) Benannte Vertragsstaaten : **CH DE FR GB LI NL**

(56) Entgegenhaltungen :
**DE-A- 2 928 348**
**DE-A- 2 951 508**
**DE-B- 2 152 016**
**DE-B- 2 626 965**
**US-A- 4 156 097**

(73) Patentinhaber : **CHEMISCHE WERKE HÜLS AG - RSP Patente / PB 15 - Postfach 13 20 D-4370 Marl 1 (DE)**

**NAARDEN INTERNATIONAL N.V. P.O.Box 2 Huizerstraatweg 28 NL-1400 CA Naarden-Bussum (NL)**

(72) Erfinder : **Kaufhold, Manfred, Dr. Jasminweg 20 D-4370 Marl (DE)**
Erfinder : **Takken, Hendrik Jan, Dr. Frans Halslaan 14 NL-1272 HN Huizen (NL)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

EP 0 095 611 B1

**Beschreibung**

Der 2-Methoxyethyl-cyclododecenylether der Formel I

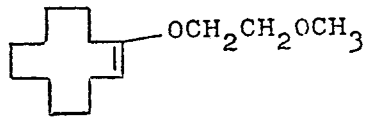

(I)

hat als geeignete Vorstufe für den entsprechenden gesättigten Ether der Formel II

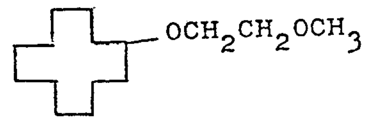

(II)

großes Interesse, da man den 2-Methoxyethyl-cyclododecylether in hoher Reinheit und mit guter Riechstoffqualität durch katalytische Hydrierung von (I) herstellen kann. Der 2-Methoxyethyl-cyclododecylether (II) dient zur Herstellung von Riechstoffkompositionen für Parfüm oder u. a. zur Parfümierung von Kosmetika (DE-OS 29 28 348 = US-PS 4 317 942).

Die Herstellung von 2-Methoxyethyl-cyclododecylether war bisher nur mit Hilfe von aufwendigen mehrstufigen Verfahren über den 2-Hydroxyethyl-cyclododecylether der Formel (III) möglich

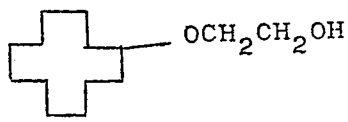

(III)

Während (III) sich noch relativ leicht herstellen läßt, beispielsweise durch Hydrierung des aus Cyclododecanon und Ethylenglykol hergestellten Ketals, ist die Veretherung dieses Alkohols nur mit großem technischen Aufwand möglich, da bei den bekannten Veretherungsverfahren (siehe Houben-Weyl), Methoden der organischen Chemie, Band VI/3, Sauerstoffverbindungen I, Teil 3, Seiten 10 bis 137 (1965) der eingesetzte Alkohol nicht vollständig umgesetzt wird. Der Restalkohol im Ether verhindert aber wegen seines unangenehmen Geruchs dessen Verwendung als Riechstoff. Eine destillative Trennung dieser Produkte ist wegen der sehr ähnlichen Siedepunkte wenig effektiv. Auch die in der DE-OS 29 28 348 vorgeschlagenen Extraktions- und Absorptionsverfahren führen nur bei sehr großem technischen Aufwand zum Ziel. Chemische Methoden kommen ebenfalls aus wirtschaftlichen Gründen nicht infrage.

Nach den Beispielen der DE-OS 29 28 348 benötigt man für die Veretherung zudem kostspielige Chemikalien, wie z. B. Natriumamid und Dimethylsulfat, deren Handhabung nicht ungefährlich ist. Es handelt sich hier um Arbeitsweisen, die im Laboratorium durchführbar sind, die aber wegen der hohen Einsatzstoffkosten und des großen technischen Aufwandes für die Sicherheit des Personals für eine technische Realisierung nicht infrage kommen.

Alle bekannten Verfahren liefern somit nur unsauberes Produkt, benötigen aufwendige technische Apparaturen oder verwenden Chemikalien, die kostspielig und wegen ihrer Handhabung nur für Arbeiten in Laboratorien geignet sind.

Es besteht daher ein großes Interesse an einem Verfahren, nach dem man in einfacher Weise bei geringem technischen Aufwand den 2-Methoxyethyl-cyclododecylether in reiner Form und guter Ausbeute herstellen kann.

Die sich hieraus ergebende Aufgabe wird erfindungsgemäß entsprechend den Angaben der Ansprüche gelöst.

Überraschenderweise erhält man durch Umsetzung von Cyclododecanon mit einem Orthoameisensäureester eines $C_1$- bis $C_4$-Alkanols und mit 2-Methoxyethanol in Gegenwart eines sauren Katalysators in einer Stufe den 2-Methoxyethyl-cyclododecenylether I in guten Ausbeuten von über 93 % und in einer Reinheit von über 99 %.

Statt in einer Stufe kann man die Synthese auch in zwei Schritten durchführen. Stellt man zunächst den Orthoameisensäureester des Methoxyethanols nach den Angaben der DE-AS 20 62 034 (= US-PS 3 903 006) her, so erhält man in der zweiten Stufe bei der Umsetzung mit Cyclododecanon nur geringe Ausbeuten. Setzt man dagegen zunächst Cyclododecanon mit einem Orthoameisensäureester eines $C_1$-bis $C_4$-Alkanols in Gegenwart eines $C_1$- bis $C_4$-Alkanols als Lösemittel um und das in der ersten Stufe erhaltene Reaktionsgemisch in einer zweiten Stufe mit 2-Methoxyethanol um, so erhält man bessere Ausbeuten, jedoch unbefriedrigende Umsätze.

Obwohl bei der Umsetzung in einer Stufe (Eintopfverfahren) wesentlich mehr Möglichkeiten für Nebenreaktionen bestehen, erhält man überraschenderweise erheblich bessere Ausbeuten bei hohen Umsätzen von 98 % und höher sowie ein reineres Produkt. Da es sich beim 2-Methoxyethyl-cyclododecenylether um eine Vorstufe für einen Riechstoff handelt, ist die Reinheit ein entscheidendes Kriterium. Daher führt man das beanspruchte Verfahren bevorzugt in einer Stufe durch.

Es ist ein weiterer Vorteil des erfindungsgemäßen Verfahrens, daß man in einfacher Weise die neue Verbindung (I) erhält, die relativ stabil ist und sich durch Destillation gut reinigen läßt.

Das erfindungsgemäße Verfahren führt man aus wirtschaftlichen Gründen bevorzugt bei Normaldruck durch, wobei die maximale Reaktionstemperatur dann die Temperatur ist, bei der das Reaktionsgemisch siedet, d. h. nach Zusammengeben der Komponenten werden die gebildeten Leichtsieder sofort unter Rühren abdestilliert. Hierbei steigert man die Reaktionstemperatur von 50 bis 250 °C, vorzugsweise von 70 bis 190 °C, insbesondere von 70 bis 160 °C, so daß ständig Destillat anfällt. Temperaturen über 250 °C sind ungeeignet, weil dann starke Zersetzung der Ameisensäureester eintritt. Vorzugsweise führt man die Umsetzung unter einem Inertgas durch, beispielsweise einer Stickstoffabdeckung.

Wenn trotz Erhöhung der Reaktionstemperatur auf 250 °C, bzw. 190 °C, bzw. 160 °C, kein Destillat mehr anfällt, senkt man den Druck auf 10 bis 25 mbar und destilliert bei diesem Druck und Temperaturen bis 190 °C, vorzugsweise bis 160 °C, weiter. Nach Beendigung der Destillation der Leichtsieder neutralisiert man den Katalysator, zweckmäßig z. B. mit 50 %iger Natronlauge und destilliert anschließend den ungesättigten Ether I (153 bis 163 °C bei 13 mbar).

Das Verfahren kann diskontinuierlich und kontinuierlich durchgeführt werden.

Die Hydrierung von I führt man in üblicher Weise bei 15 bis 300 bar Wasserstoffdruck mit einem Palladium- oder Nickelkatalysator bei erhöhter Temperatur, beispielsweise 50 bis 100 °C durch.

Man erhält den 2-Methoxyethyl-cyclododecylether (II) mit einer Reinheit von über 99 % und guter Riechstoffqualität.

Als Orthoameisensäureester (IV) kann man bei dem erfindungsgemäßen Verfahren Ester von Alkoholen einsetzen, die niedriger sieden als 2-Methoxyethanol, d. h. unter 124 °C

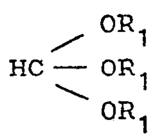

$R_1$ = $C_1$- bis $C_4$-Alkylreste (IV)

Bevorzugt setzt man die marktgängigen Orthoameisensäuretrimethylester und -triethylester ein, von denen der Trimethylester die besten Ausbeuten liefert.

Als Katalysatoren sind Säuren wie Chlorwasserstoff, Schwefelsäure und Phosphorsäure sowie typische Friedel-Crafts-Katalysatoren wie Chloride des Eisens, Zinks, Zinns und Aluminiums geeignet. Die besten Ausbeuten und die höchste Reinheit von I erhält man bei Verwendung von Alkylbenzolsulfonsäuren, wie z. B. p-Toluolsulfonsäure und $C_{10}$- bis $C_{13}$-Alkylbenzolsulfonsäure. Die zuletzt genannte Säure garantiert als Katalysator einen besonders niedrigen Schwefelgehalt des ungesättigten Ethers I. Dies ist für die Lebensdauer des Hydrierkontakts wichtig. Den sauren Katalysator setzt man in Mengen von 0,2 bis 1 g, vorzugsweise 0,3 bis 0,5 g, insbesondere etwa 0,35 g, bezogen auf 100 g eingesetztes Keton, ein.

Als Lösungsmittel sind $C_1$- bis $C_4$-Alkanole geeignet. Sie sind nur beim 2-Stufenverfahren erforderlich.

Der erhaltene 2-Methoxyethylcyclododecenylether ist eine farblose bis schwach gelbliche, ölige Flüssigkeit mit einem charakteristichen grünen, an frisches Gras erinnernden Geruch, er hat bei 13 mbar einen relativ breiten Siedebereich von ca. 153 °C bis 163 °C, weil er in zwei Isomeren vorliegt. Nach gaschromatographischer Analyse enthält das Destillat zwei Komponenten, beispielsweise in Konzentrationen von 20,0 und 79,8 %.

2-Methoxyethyl-cyclododecenylether verwendet man insbesondere zur Herstellung des 2-Methoxyethyl-cyclodecylether (II), einem wertvollen Riechstoff, durch katalytische Hydrierung, beispielsweise über Nickel- oder Palladium-Katalysatoren bei einem erhöhten Druck von 15 bis 300 bar und Temperaturen von 50 bis 100 °C, wobei man den 2-Methoxyethyl-cyclododecylether in hoher Reinheit und guter Riechstoffqualität erhält. Der 2-Methoxyethyl-cyclododecylether (II) dient zur Herstellung von Riechstoffkompositionen, insbesondere für Riechstoffkombinationen mit typisch holzartiger Geruchsnote.

## Beispiel 1

Man benutzt eine Glasapparatur, die aus einem Dreihalskolben mit Rührer, Thermometer und einer Destillationskolonne mit passendem Aufsatz, Rückflußeinsteller, Kühler, Vorlage usw. besteht.

Man setzt 541,4 g (= 5 Mol) Orthoameisensäuretrimethylester (98 %ig), 1 141,5 g (= 15 Mol) 2-Methoxyethanol, 911,5 g (= 5 Mol) Cyclododecanon und 3,1 g $C_{10}$- bis $C_{13}$-n-Alkylbenzolsulfonsäure ein und erhitzt das Gemisch unter Rühren und Stickstoffabdeckung am Rückfluß zum Sieden. Die bei der Umsetzung entstehenden niedrig siedenden Komponenten destilliert man bei Normaldruck bzw. bei 16 mbar, wie in folgender Tabelle angegeben, ab.

Wenn bei der Fraktion 3 der Destillatanfall aufhört, stellt man einen Druck von 16 mbar ein, erhöht die Temperatur im Sumpf wieder auf 160 °C und destilliert das restliche 2-Methoxyethanol ab. Danach kühlt man ab und gibt zur Neutralisation des Katalysators 5 ml 50 %ige Natronlauge zu. Nach Zusatz von 0,5 g

Soda unterwirft man das Reaktionsprodukt ohne weitere Aufarbeitung einer fraktionierten Destillation. In einem Siedebereich von 153 bis 163 °C bei 13 mbar erhält man 1 121 g 2-Methoxyethyl-cyclododecenylether mit einer gaschromatographisch ermittelten Reinheit von 99,8 %. Die Ausbeute auf den Einsatz bezogen beträgt 93,2 % d. Th. Der Chlor- und Schwefelgehalt liegen bei je 1 ppm.

Der 2-Methoxyethyl-cyclododecenylether ist eine farblose bis schwach gelbliche, ölige Flüssigkeit mit grünem, an frisches Gras erinnernden Geruch. Nach gaschromatographischer Analyse liegen zwei Isomere in Konzentrationen von 20,0 und 79,8 % vor, deren aus dem Chromatogramm berechneten Siedepunkte bei 310 bzw. 324 °C liegen.

Das 1 H-NMR-Spektrum zeigt folgende charakteristische Lage der Protonen :

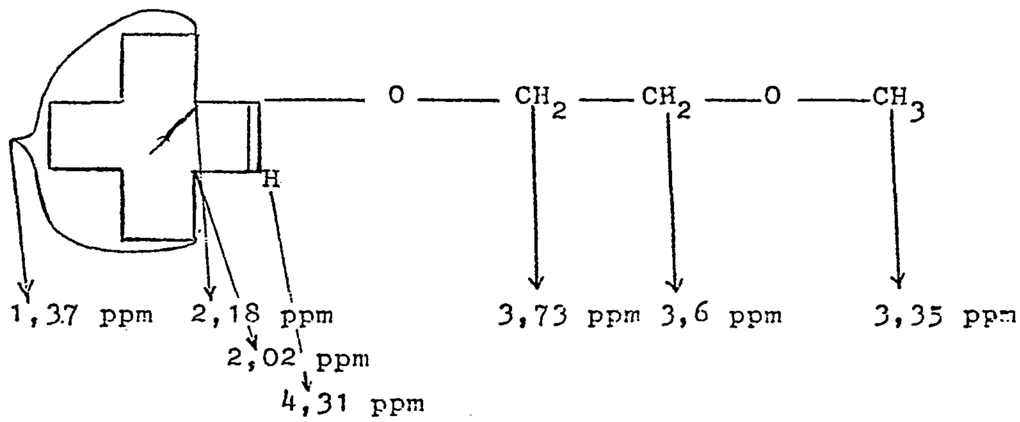


(Siehe Tabelle Seite 5 f.)

| Fraktion Nr. | Druck mbar | Siedebereiche in °C im Sumpf | Kopf | Menge g | Verhältnis Rücklauf zu Abnahme | Produkt |
|---|---|---|---|---|---|---|
| 1 | 1 013 | 70 bis 96 | 32 bis 48 | 296 | 10 : 1 | Methylformiat |
| 2 | 1 013 | 98 bis 128 | 62 bis 64 | 309 | 5 : 1 | Methanol |
| 3 | 1 013 | 132 bis 160 | 119 bis 122 | 777 | 5 : 1 | 2-Methoxyethanol |
| | 16 | 150 bis 160 | 38 | | | |

Beispiel 2

Die Umsetzung erfolgt nach den Angaben des Beispiels 1, aber in Gegenwart von 6,5 g p-Toluolsulfonsäure anstelle von 3,1 g $C_{10}$- bis $C_{13}$-n-Alkylbenzolsulfonsäure. Man erhält in gleicher Ausbeute den 2-Methoxyethylcyclododecenylether, jedoch mit einem Schwefelgehalt von 8 ppm. Der Chlorgehalt liegt bei 1 ppm. Diesen höheren Schwefelgehalt kann man in üblicher Weise, beispielsweise mit Hilfe eines Nickelkatalysators auf < 1 ppm S erniedrigen.

Beispiel 3

Die Umsetzung erfolgt in der im Beispiel 1 angegebenen Apparatur.

Man setzt 364,6 g (= 2 Mol) Cyclododecanon, 212,2 g (= 2 Mol) Orthoameisensäuretrimethylester, 64,1 g (= 2 Mol) Methanol und 1,1 g $C_{10}$- bis $C_{13}$-Alkylbenzolsulfonsäure ein.

Dieses Gemisch erhitzt man unter Rühren auf über 70 °C bis Destillat anfällt. Das gebildete Methylformiat wird, wie im Beispiel 1 angegeben, abdestilliert, bis die Siedetemperatur des Methanols erreicht ist. Dann kühlt man ab, um die 2. Synthesestufe durchzuführen.

Man setzt nun 456,6 (= 6 Mol) 2-Methoxyethanol zu, erhitzt wieder unter Rühren bis zum Sieden und destilliert die leichtsiedenden Komponenten, zunächst Methanol, ab. Die weitere Durchführung erfolgt wie im Beispiel 1 beschrieben.

Ausbeute 70,5 % Reinheit 99,2 %.

Beispiel 4

In einem 100 ml-Reaktor hydriert man den 2-Methoxyethylcyclododecenylether mit einem Einsatz von 20 ml pro Stunde bei 300 bar Wasserstoffdruck und 50 °C über einem Palladium-Träger-Kontakt in bekannter Weise. Man erhält den 2-Methoxyethyl-cyclododecylether (II) in praktisch quantitativer Ausbeute und einer gaschromatographisch ermittelten Reinheit von über 98,5 %. Durch Destillation erhält man in über 95 %iger Ausbeute den 2-Methoxyethyl-cyclododecylether mit guter Riechstoffqualität.

Senkt man bei der Hydrierung den Druck von 300 auf 15 bar und erhöht die Temperatur von 50 auf 80 °C, so erhält man praktisch die gleichen Ergebnisse.

**Ansprüche**

1. 2-Methoxyethyl-cyclododecenylether der Formel I

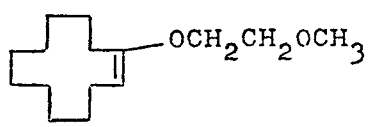

(I)

2. Verfahren zur Herstellung von 2-Methoxyethyl-cyclododecenylether, dadurch gekennzeichnet, daß man 2-Methoxyethanol und einen Orthoameisensäureester der allgemeinen Formel (IV)

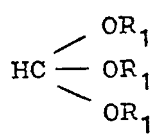

(IV)

in der $R_1C_1$- bis $C_4$-Alkylreste bedeuten, bei Temperaturen von 50 bis 250 °C in Gegenwart eines sauren Katalysators mit Cyclododecanon, gegebenenfalls in Gegenwart eines $C_1$- bis $C_4$-Alkanols als Lösemittel, umsetzt und die dabei entstehenden leichtsiedenden Verbindungen abdestilliert.

3. Verfahren nach Anspruch 2, dadurch gekennzeichnet, daß man als saure Katalysatoren Alkylbenzolsulfonsäuren, wie p-Toluolsulfonsäure oder $C_{10}$- bis $C_{13}$-n-Alkylbenzolsulfonsäure einsetzt.

4. Verfahren nach Anspruch 2 und 3, dadurch gekennzeichnet, daß man die Umsetzung bei Temperaturen von 70 bis 190 °C, vorzugsweise von 70 bis 160 °C, durchführt.

5. Verfahren nach den Ansprüchen 2 bis 4, dadurch gekennzeichnet, daß man die leichtsiedenden Verbindungen zunächst bei Normaldruck und der Reaktionstemperatur abdestilliert und anschließend die restlichen Leichtsieder bei einem Druck von 10 bis 25 mbar und Temperaturen bis 190 °C, vorzugsweise bis 160 °C, abdestilliert.

6. Verwendung des 2-Methoxyethyl-cyclododecenylethers für die katalytische Hydrierung zum 2-Methoxyethylcyclodecylether (II).

**Claims**

1. 2-Methoxyethyl cyclododecenyl ether of the formula I

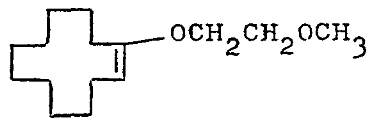

(I)

2. Process for the preparation of 2-methoxyethyl cyclododecenyl ether, characterised in that 2-methoxyethanol and an orthoformic acid ester of the general formula (IV)

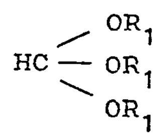

(IV)

in which the $R_1$'s denote $C_1$-$C_4$-alkyl radicals, are reacted at temperatures of 50 to 250 °C, in presence of an acid catalyst, with cyclododecanone, if appropriate in the presence of a $C_1$-$C_4$-alkanol as the solvent, and the low-boiling compounds thereby produced are distilled off.

3. Process according to Claim 2, characterised in that alkylbenzenesulphonic acids, such as p-toluenesulphonic acid or a $C_{10}$-$C_{13}$-n-alkylbenzenesulphonic acid, are employed.

4. Process according to Claim 2 and 3, characterised in that the reaction is carried out at temperatures of 70 to 190 °C, preferably of 70 to 160 °C.

5. Process according to Claims 2 to 4, characterised in that the low-boiling compounds are distilled off initially under normal pressure and at the reaction temperature, and thereafter the residual low-boilers are distilled off at a pressure of 10 to 25 mbar and at temperatures of up to 190 °C, preferably up to 160 °C.

6. Use of the 2-methoxyethyl cyclododecenyl ether for catalytic hydrogenation to give 2-methoxyethyl cyclododecyl ether (II).

**Revendications**

1. L'éther 2-méthoxy-éthyl-cyclododécénylique de la formule I

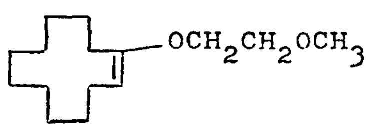

(I)

2. Procédé de préparation de l'éther 2-méthoxy-éthylcyclododécénylique, caractérisé par le fait qu'à des températures de 50 à 250 °C et en présence d'un catalyseur acide, on fait réagir sur de la cyclododécanone, le cas échéant en présence d'un alcanol en $C_1$ à $C_4$ en tant que solvant, du 2-méthoxy-éthanol et un ester de l'acide orthoformique répondant à la formule générale (IV)

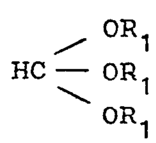

(IV)

dans laquelle $R_1$ représente des restes alkyle en $C_1$ à $C_4$, et qu'on élimine par distillation les composés à bas point d'ébullition qui prennent alors naissance.

3. Procédé selon la revendication 2, caractérisé par le fait qu'on utilise, comme catalyseurs acides, des acides alkyl-benzène-sulfoniques comme l'acide p-toluène-sulfonique ou un acide n-alkylbenzène-sulfonique en $C_{10}$ à $C_{13}$.

.4. Procédé selon les revendications 2 et 3, caractérisé par le fait qu'on effectue la réaction à des températures de 70 à 190 °C, de préférence de 70 à 160 °C.

5. Procédé selon les revendications 2 à 4, caractérisé par le fait qu'on élimine d'abord par distillation les composés à bas point d'ébullition sous la pression normale et à la température de la réaction et qu'on élimine ensuite par distillation les fractions restantes à bas point d'ébullition sous une pression de 10 à 25 mbar et à des températures de 190 °C, de préférence jusqu'à une température de 160 °C.

6. L'utilisation de l'éther 2-méthoxy-éthyl-cyclododécénylique pour l'hydrogénation catalytique en éther 2-méthoxy-éthyl-cyclododécylique (II).